# EUROPEAN PATENT SPECIFICATION

(11) **EP 0 945 075 B1**
(45) Date of publication and mention of the grant of the patent: **28.01.2004**
(21) Application number: 99830153.5
(22) Date of filing: 19.03.1999
(51) Int. Cl.: A23L 1/30, A23L 1/09, A23L 1/03

(54) **Nutritional supplement**
Nahrungsergänzungsmittel
Supplément d'aliments

(30) Priority: 25.03.1998 MT 130698
(43) Date of publication of application: 29.09.1999
(73) Proprietor: Garuti, Eliseo, 33312 Valdedios-Villaviciosa, Asturias (ES)
(72) Inventor: Garuti, Eliseo, 33312 Valdedios-Villaviciosa, Asturias (ES)
(74) Representative: Martini, Lazzaro

(56) References cited:
- EP-A- 0 634 109
- WO-A-93/17589
- WO-A-95/28084
- DATABASE WPI Section Ch, Week 8709 Derwent Publications Ltd., London, GB; Class A96, AN 87-059059 XP002111006 & JP 61 207322 A (SUNSTAR KK), 13 September 1986 (1986-09-13)

## Description

The present invention relates to an alimentary integrator which results particularly useful in degenerative forms, in weakening of the immune system and in stepped-up organic deterioration.

It is a well-known fact that atmospheric and environmental (including electro-magnetic) pollution, radiation in general, an irregular diet (with an excess of peroxidated food fats) and food alternations themselves (preservatives, genetically modified substances, etc,) ciagarette smoke, prolungated emotional stress and immune and infallamtory reactions are a continual source of free radicals which cause damage to the organism on the cellular level by way of chemical oxidizations of lipids and proteins (oxidated stress). The same angiogenesis, triggering off a hyperoxygenation of the new cytoplasmatic capillary vessels is itself a source of oxidizing stress. The general effects thus produced in the organism are mainly the following: a weakening of the immune system; a stepped-up physiological organic deterioration; the production of degenerative forms.

In this way biological, physical and chemical alterations are formed that can cause damage to the cellular membrane and to the DNA.

In fact, in the presence of oxidizing stress, the NA + - K + pump does not perform its task correctly and there occurs an imbalance of the four fundamental cations which are needed to maintain cellular functioning, namely sodium, potassium, calcium and magnesium. Especially in the presence of degenerative diseases and in particular neoplastic forms, Sodium NA + (a prevalent cation in extracellular fluids) tends to substitute Potassium K + inside the cell with a resulting serious imbalance and alteration of the NA - K pump. WO 95/28084 discloses a dietery ascorbic acid supplement composition for providing human immune system activity.

Aim of the present invention is to eliminate the above mentioned drawbacks providing with an alimentary integrator having the characteristics of claim 1. Other characteristics are object of the dependent claims.

Among the advantages of the present invention there is that the integrator is a very powerful anti-oxidizing that reduces the effect of free radicals; that it strengthens the activity of the immune system mantaining or revitalizing the concentration of intracellular potassium to the required levels; that it ties together the functional characteristics of its components giving rise to results surprisingly better in respect to the components singularly used; that it is completely atoxyic (at prescribed doses); that it can be used over a long period of time; that it is a very simple posology.

The alimentary integrator of the invention is constitued by a compound of Potassium Bicarbonate (KHCO₃) and an anti-oxidant agent that comprises D Ribose (C₅H₁₀O₅) and that can comprise Ascorbic Acid L (C₆H₈O₆).

In a possible embodiment, the integrator is constitued by a salt derived from Ascorbic Acid L, and is obtained by uniting Potassium Bicarbonate, Ascorbic Acid L and D Ribose in the purest crystallized form. The compound thus obtained, soluble in water, takes the name of Potassium Ribose Ascorbate.

Ascorbic Acid salifies easily with alkaline bicarbonates dissolved in cold distilled water and with warm earthy-alkaline carbonates at a temperature of 45 - 50 °C, and working out of CO₂. By cold evaporation, under high vacuum, crystallized salts (ascorbates) are obtained.

Potassium Ribose Ascorbate is a pure microcrystalline easily water-soluble white salt and somewhat unstable due to its easy oxidizability. It is secured through the salification of Ascorbic Acid L and D Ribose in cold water solution with Potassium Bicarbonate. Its action does not produce any toxicity (given the stated dosages) and can be used for a long time. Biologically it follows the pattern of Ascorbic Acid.

Potassium Ribose Ascorbate, as an Ascorbic Acid derivate, it can take on two isomeric configurations: the oenolic form and the furanoid form; when in solution it assumes the latter form.

Potassium Ribose Ascorbate that, as expressed above, is a very powerful anti-oxidizing, completely atoxyic that can be used over a long period of time (at prescribed doses), acts in a way that reduces the effect of free radicals, strengthening the activity of the immune system and maintaining or revitalizing the concentration of intracellular potassium to the required levels. It is therefore a most valid alimentary integrator and a very powerful cellular anti-oxidizer which ties together the functional characteristics of Ascorbic Acid L and of Potassium, manifesting itself as more active than the two constituents separately taken. Moreover, the presence of D Ribose, for its charecteristics of a molecule structurally present in the intimate architecture of the cell, makes the compound more effective.

Blood haemoglobin contains pyrrolic rings in its molecule; equally pyrrole derivates must be considered many fundamental amino acids. Also of note is the fact that black pigments, skin, hair, moles or birth-marks, etc are closely related to pyrrole black spots, which allows the assumption that said pigments are oxidized compounds and polycondensed compounds having a pyrrolic structure.

Pyrrole, Thiophene and Furan are similar to each other; in the formation of their compounds they follow Angeli's rule of analogies. It is therefore reasonable to assume that during the biological synthesis process of protein derivatives from such compounds there may occur analogous chemical and physiological reactions and that under particular conditions a pyrrolic group may be replaced by a similar thiophenic or furanoid group.

It is important to note that the hydrogen present in the pyrrolic structure can be easily replaced by the potassium cation but not by the sodium potion (the Camician effect), regardless of the physical and chemical affinity between these two.

Both potassium haemoglobinate and potassium proteinate contain pyrrolic structures which can be salified with KHCO₃; the Potassium Ribose Ascorbate contains in its molecule a furanosic group which by analogy may replace one of the pyrrolic groups of potassium haemoglobinate and proteinate.

This is of the utmost importance because at the beginning of a degenerative process, particularly of neoplastic type, the pyrrolic group are inactive and it is quite likely that the starting point of a degenerative disease is the peptide molecule containing pyrrolic groups at the RNA level. It is assumed that the opening of the pyrrolic molecule (the Camician effect) may give rise to a triggering off of RNA polymerization, this being the beginning of the biological phase of a neoplasia.

Therefore Potassium Ribose Ascorbate, in reactivating the pyrrolic group, restores the structuring phenomena of the cellular auto-synthesis to the required physiological normality.

Besides, since salification is a reversible process, Potassium Ribose Ascorbate, carried by haemoglobin and introduced into the cell, re-establishes the equilibrium amongst the intermolecular forces of the peptide groups which are present inside the cell membrane and brings back (or maintains) the required levels of the intracellular potassium concentration.

Finally, Ascorbate Acid L residing in Potassium Ribose Ascorbate is fully capable of carrying out its functions, protecting above all the cell from the effects of free radicals, precisely by virtue of its anti-oxidizing characteristics, while D Ribose, as previously pointed out, amplifies the effect of the compound.

Potassium Ribose Ascorbate is especially active against degenerative disease since, as explained earlier, when confronted by a degenerative process, it corrects the process of cellular metabolism by inhibiting its progress and giving it back the equilibrium of its functions with satisfactory results.

It emerges from stoichiometric calculations that the proportion between potassium bicarbonate and Ascobric Acid L must be of a 2 - 1 relation. In particular conditions, it is possible to double the amount of potassium bicarbonate, determining a 4 - 1 relation.

Regarding the storage and the dosage, the Potassium Ribose Ascorbate must be protected from humidity and the sun's rays and preserved separately in bags, phials or other suitable containers for preparing an extemporary solution of very pure Ascorbic Acid L, D Ribose and Bicarbonate of Potassium according to the proportions deriving from the experimental tests and of which is described an example.

Each extemporary preparation comprises a bag or a phial of Ascorbic Acid L containing a dose of same, a bag or a phial of Potassium Bicarbonate containing two doses of same and a bag or a phial of D Ribose containing an amount between 0.1% and 10%, preferably 2%, of the inclusive dosage of Potassium Ascorbate. In the bag the contents are crystallized, in the phial the contents are in solution of bidistilled water.

In the administering of Potassium Ribose Ascorbate as a therapy against degenerative diseases, the contents of a dose of Ascorbic Acid L, D Ribose (2%) and two doses of Potassium bicarbonate can be blended in order to obtain the cold extemporary solution of Potassium Ribose Ascorbate to be admistered, for example, three times a day.

Experimentally, it is established that the general course of action above described may undergo variations due to particular physiopathological conditions, according to medical evaluation, for instance, by doubling the Potassium bicarbonate dosage (determining the 4-1 relation between KHCO₃ e C₆H₈O₆ above mentioned) or by reducing the administration of the medication to twice per day; at any rate, the maximum dosage of Potassium Ribose Ascorbate should not exceed 3 g daily.

At the preventive level, Potassium Ribose Ascorbate can be used at the same dosages of therapy but is can be administered only once a day, on alternate days.

For children, mantaining the same dosages, the preventive administration changes with the age.

In another possible embodiment, the compound of the present invention is a salt derived from D Ribose and it is obtained by uniting Potassium Bicarbonate and D Ribose in the purest crystallized form. The compound thus obtained, soluble in water, takes the name of Potassium Ribosate.

It is particularly effective in degenerative forms for people allergic to Ascorbic Acid L and to ascorbate (in particular to Potassium Ascorbate).

It is known that Ascorbic Acid L and D Ribose are molecules of similar structure, and belong with the same functional group in which the hdyroxil 0 - H in the reaction affected in the Potassium Ribose Ascorbate formation is substituted by the O - K group.

In this way, it can be deemed as a substratum or the precursor of another in biochemical reactions.

Therefore, in pathologic situations of allergy to Ascorbic Acid L, D Ribose is a fundamental substitute, with similar functional characteristics.

Ribose salifies easily with alkaline bicarbonates dissolved in cold distilled water and with warm earthy-alkaline carbonates at a temperature of 45 - 50 °C, and working out of CO₂, for its structural and functional characteristics similar to Ascorbic Acid L. By cold evaporation, under high vacuum, crystallized salts (ribosates) are obtained.

Potassium Ribosate is a pure microcrystalline easily water-soluble white salt and somewhat unstable due to its easy oxidizability. It is secured through the salification of D Ribose in cold water solution with Potassium bicarbonate. Its action does not produce any toxicity (given the stated dosages) and can be used for a long time. Biologically it follows the pattern of Ascorbic Acid.

Potassium Ribosate, as an Ascorbic Acid precursor, it can take on two isomeric configurations: the oenolic form and the furanoid form; when in solution it assumes the latter form.

Also Potassium Ribosate, similarly to Potassium Ribose Ascorbate, is a very powerful anti-oxidizing, completely atoxyic that can be used over a long period of time (at prescribed doses), acts in a way that reduces the effect of free radicals, strengthening the activity of the immune system and maintaining or revitalizing the concentration of intracellular potassium to the required levels. It is therefore a most valid alimentary integrator and a very powerful cellular anti-oxidizer which ties together the functional characteristics of D Ribose and of Potassium, manifesting itself as more active than the two constituents separatety taken.

As said about Potassium Ribose Ascorbate, blood haemoglobin contains pyrrolic rings in its molecule; equally pyrrole derivates must be considered many fundamental amino acids. Also of note is the fact that black pigments, skin, hair, moles or birth-marks, etc are closely related to pyrrole black spots, which allows the assumption that said pigments are oxidized compounds and polycondensed compounds having a pyrrolic structure.

Pyrrole, Thiophene and Furan are similar to each other; in the formation of their compounds they follow Angeli's rule of analogies. It is therefore reasonable to assume that during the biological synthesis process of protein derivatives from such compounds there may occur analogous chemical and physiological reactions and that under particular conditions a pyrrolic group may be replaced by a similar thiophenic or furanoid group.

It is important to note that the hydrogen present in the pyrrolic structure can be easily replaced by the potassium cation but not by the sodium potion (the Camician effect), regardless of the physical and chemical affinity between these two.

Both potassium haemoglobinate and potassium proteinate contain pyrrolic structures which can be salified with KHCO₃; the Potassium Ribose Ascorbate contains in its molecule a furanosic group which by analogy may replace one of the pyrrolic groups of potassium haemoglobinate and proteinate.

This is of the utmost importance because at the beginning of a degenerative process, particularly of neoplastic type, the pyrrolic group are inactive and it is quite likely that the starting point of a degenerative disease is the peptide molecule containing pyrrolic groups at the RNA level. It is assumed that the opening of the pyrrolic molecule (the Camician effect) may give rise to a triggering off of RNA polymerization, this being the beginning of the biological phase of a neoplasia.

Therefore Potassium Ribosate, in reactivating the pyrrolic group, restores the structuring phenomena of the cellular auto-synthesis to the required physiological normality.

Besides, since salification is a reversible process, Potassium Ribosate, carried by haemoglobin and introduced into the cell, re-establishes the equilibrium amongst the intermolecular forces of the peptide groups which are present inside the cell membrane and brings back (or maintains) the required levels of the intracellular potassium concentration.

Finally, the compound Potassium Ribosate protects the cell from the effects of free radicals, precisely by virtue of its anti-oxidizing characteristics.

Potassium Ribosate is especially active against degenerative disease since, as explained earlier, when confronted by a degenerative process, it corrects the process of cellular metabolism by inhibiting its progress and giving it back the equilibrium of its functions with satisfactory results.

It emerges from stoichiometric calculations that the proportion between Potassium Bicarbonate and Ribose must be of a 2 - 1 relation, but it is possible a 3-1 relation.

Regarding the storage and the dosage, the compund of Potassium Ribosate must be protected from humidity and the sun's rays and preserved separately in bags, phials or other suitable containers for preparing an extemporary solution of very pure Ascorbic Acid L, D Ribose and Bicarbonate of Potassium according to the following preferable proportions. The proportions provide to use a bag or a phial of Potassium bicarbonate containing two doses of same and a bag or a phial of D Ribose containing a dose of same. It is possible to use three doses of Potassium Bicarbonate and a dose of D Ribose.

In the administering of Potassium Ribosate as a therapy against degenerative diseases, the contents of a dose of Ribose and two doses of Potassium bicarbonate can be blended in order to obtain the cold extemporary solution of Potassium Ribosate to be admistered, for example, three times a day.

Similarly to Potassium Ribosate Ascorbate, the above described general course of action of Potassium Ribosate may undergo variations due to particular physiopathological conditions, according to medical evaluation, for instance, by doubling the Potassium bicarbonate dosage (determining a 4-1 relation between Potassium Bicarbonate and D Ribose) or by reducing the daily administration of the medication; at any rate, the maximum dosage of Potassium Ribosate should not exceed 2 g daily. At the preventive level and in the administration to childre, Potassium Ribosate can be used similarly to Potassium Ribose Ascorbate.

## Claims

1. Alimentary integrator **characterized by** the fact that it is constitued by a compound of Potassium Bicarbonate and an anti-oxidant agent comprising D-Ribose.

2. Alimentary integrator according to claim 1, **characterized by** the fact that said anti-oxidant agent comprises D-Ribose and Ascorbic Acid L.

3. Alimentary integrator according to claim 1, **characterized by** the fact that comprises two parts of Potassium Bicarbonate and one part of D-Ribose.

4. Alimentary integrator according to claim 1, **characterized by** the fact that comprises three parts of Potassium Bicarbonate and one part of D-Ribose.

5. Alimentary integrator according to claim 1, **characterized by** the fact that comprises four parts of Potassium Bicarbonate and one part of D-Ribose.

6. Alimentary integrator according to claim 2, **characterized by** the fact that comprises one part by weight of Ascorbic Acid L every two parts of Potassium Bicarbonate and that the amount of D-Ribose is comprised between 0.1 % and 10% by weight of the sum of the other components.

7. Alimentary integrator according to claim 2, **characterized by** the fact that comprises one part by weight of Ascorbic Acid L every four parts of Potassium Bicarbonate and that the amount of D-Ribose is comprised between 0.1% and 10% by weight of the sum of the other components.

8. Alimentary integrator according to claim 6 or 7, **characterized by** the fact that the amount of D-Ribose is 2% by weight of the sum of the other components.

9. Alimentary integrator according to any one of the previous claims, **characterized by** the fact that the components of said compound are in single doses for each component, associable at the time of use, in order to obtain an extemporary solution of the integrator.

## Patentansprüche

1. Nahrungsmittel-Integrator, **dadurch gekennzeichnet, daß** er von einer Zusammensetzung aus Kaliumbicarbonat und einem Anti-Oxidationsmittel gebildet wird, das D-Ribose umfaßt.

2. Nahrungsmittel-Integrator nach Anspruch 1, **dadurch gekennzeichnet, daß** das Anti-Oxidationsmittel D-Ribose und Ascorbinsäure L enthält.

3. Nahrungsmittel-Integrator nach Anspruch 1, **dadurch gekennzeichnet, daß** er zwei Teile Kaliumbicarbonat und ein Teil D-Ribose enthält.

4. Nahrungsmittel-Integrator nach Anspruch 1, **dadurch gekennzeichnet, daß** er drei Teile Kaliumbicarbonat und ein Teil D-Ribose enthält.

5. Nahrungsmittel-Integrator nach Anspruch 1, **dadurch gekennzeichnet, daß** er vier Teile Kaliumbicarbonat und ein Teil D-Ribose enthält.

6. Nahrungsmittel-Integrator nach Anspruch 2, **dadurch gekennzeichnet, daß** er ein Gewichtsteil Ascorbinsäure L für jeweils zwei Teile Kaliumbicarbonat enthält, und daß die Menge von D-Ribose zwischen 0,1 Gew.-% und 10 Gew.-% der Summe der anderen Bestandteile liegt.

7. Nahrungsmittel-Integrator nach Anspruch 2, **dadurch gekennzeichnet, daß** er ein Gewichtsteil Ascorbinsäure L für jeweils vier Teile Kaliumbicarbonat enthält, und daß die Menge der D-Ribose zwischen 0,1 Gew.-% und 10 Gew.-% der Summe der anderen Bestandteile liegt.

8. Nahrungsmittel-Integrator nach Anspruch 6 oder 7, **dadurch gekennzeichnet, daß** die Menge von D-Ribose 2 Gew.-% der Summe der anderen Bestandteile beträgt.

9. Nahrungsmittel-Integrator nach einem der vorhergehenden Ansprüche, **dadurch gekennzeichnet, daß** die Bestandteile der Zusammensetzung in einzelnen Dosen für jeden Bestandteil vorliegen, die zum Zeitpunkt der Verwendung zusammenfügbar sind, um eine frisch hergestellte Lösung des Integrators zu erhalten.

## Revendications

1. Intégrateur alimentaire **caractérisé par le fait qu'**il est constitué d'un composé de bicarbonate de potassium et d'un agent anti-oxydant comprenant le D-ribose.

2. Intégrateur alimentaire selon la revendication 1, **caractérisé par le fait que** ledit agent antioxydant comprend le D-ribose et l'acide L ascorbique.

3. Intégrateur alimentaire selon la revendication 1, **caractérisé par le fait qu'**il comprend deux parties de bicarbonate de potassium et une partie de D-ribose.

4. Intégrateur alimentaire selon la revendication 1, **caractérisé par le fait qu'**il comprend trois parties de bicarbonate de potassium et une partie de D-ribose.

5. Intégrateur alimentaire selon la revendication 1, **caractérisé par le fait qu'**il comprend quatre parties de bicarbonate de potassium et une partie de D-ribose.

6. Intégrateur alimentaire selon la revendication 2, **caractérisé par le fait qu'**il comprend une partie en poids d'acide L ascorbique chaque deux parties de bicarbonate de potassium et que la quantité de D-ribose est comprise entre 0,1 et 10 % en poids de la somme des autres composants.

7. Intégrateur alimentaire selon la revendication 2, **caractérisé par le fait qu'**il comprend une partie en poids d'acide L ascorbique chaque quatre parties de bicarbonate de potassium et que la quantité de D-ribose est comprise entre 0,1 et 10 % en poids de la somme des autres composants.

8. Intégrateur alimentaire selon la revendication 6 ou 7, **caractérisé par le fait que** la quantité de D-ribose est de 2 % en poids de la somme des autres composants.

9. Intégrateur alimentaire selon l'une quelconque des revendications précédentes, **caractérisé par le fait que** les composants dudit composé sont sous la forme de dose unitaire pour chaque composant, associables au moment de l'usage, pour obtenir une solution extemporanée de l'intégrateur.
